(19) Europäisches Patentamt
European Patent Office
Office européen des brevets

(11) Numéro de publication: **0 130 878**
**A1**

(12) **DEMANDE DE BREVET EUROPEEN**

(21) Numéro de dépôt: **84401255.9**

(22) Date de dépôt: **18.06.84**

(51) Int. Cl.⁴: **C 07 D 471/16**, A 61 K 31/435 // C07D209/16 ,(C07D471/16, 221/00, 221/00, 209/00)

(30) Priorité: **05.07.83 FR 8311153**

(43) Date de publication de la demande: **09.01.85 Bulletin 85/2**

(84) Etats contractants désignés: **AT BE CH DE FR GB IT LI LU NL SE**

(71) Demandeur: **SYNTHELABO, 58, rue de la Glacière, F-75621 Paris Cedex 13 (FR)**

(72) Inventeur: **Bertin, Jean, 55, rue d'Estienne d'Orves, F-92140 Clamart (FR)**
Inventeur: **Frost, Jonathan, 7, Route de Montjean, F-91320 Wissous (FR)**
Inventeur: **Wick, Alexander, 10, Boulevard des Plants, F-78860 Saint Nom la Breteche (FR)**

(74) Mandataire: **Ludwig, Jacques et al, SYNTHELABO Service Brevets 58, rue de la Glacière, F-75621 Paris Cedex 13 (FR)**

(54) **Dérivés d'hexahydro-indolo-naphtyridines, leur préparation et leur application en thérapeutique.**

(57) Dérivés d'héxahydro-indolo-naphtyridines, sous la forme de racémates ou d'énantiomères, répondant à la formule (I)

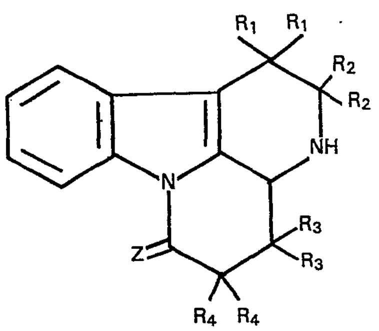

(I)

dans laquelle

Z représente un atome d'oxygène ou 2 atomes d'hydrogène,

$R_1$ ou $R_2$ ou $R_3$ ou $R_4$ représente le radical méthyle tandis que les autres sont des atomes d'hydrogène, ainsi que leurs sels d'addition aux acides pharmaceutiquement acceptables.

Application en thérapeutique.

EP 0 130 878 A1

DERIVES D'HEXAHYDRO-INDOLO-NAPHTYRIDINES LEUR PREPARATION ET LEUR APPLICATION EN THERAPEUTIQUE.

La présente invention concerne des dérivés d'hexahydro-indolo-naphtyridines, leur préparation et leur application en thérapeutique.

Les composés de l'invention répondent à la formule (I)

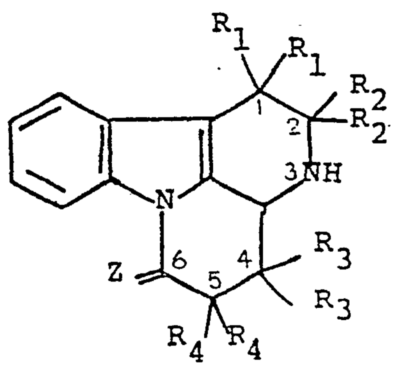

(I)

dans laquelle
Z représente un atome d'oxygène ou 2 atomes d'hydrogène,
$R_1$ ou $R_2$ ou $R_3$ ou $R_4$ représente le radical méthyle tandis que les autres sont des atomes d'hydrogène.

Les sels d'addition que forment les composés avec les acides pharmaceutiquement acceptables font partie de l'invention. Les composés de l'invention sont obtenus sous la forme de racémates. Les énantiomères des composés (I) font partie de l'invention.

Les composés préférés sont ceux dans la formule desquels Z représente deux atomes d'hydrogène.

Selon l'invention, on peut préparer les composés (I) selon les schémas réactionnels indiqués ci-dessous.

Schéma 1

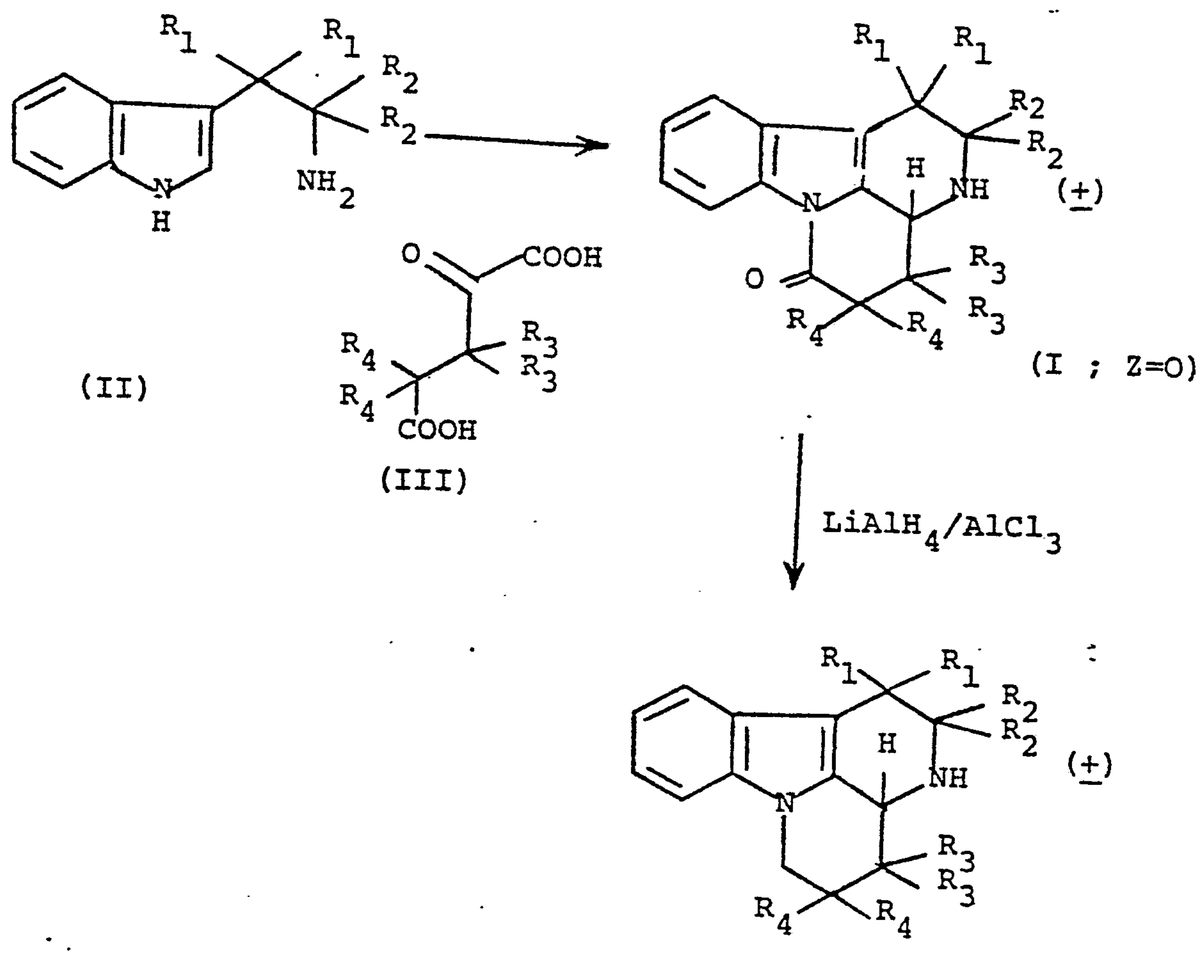


(II) (III) (I ; Z=O) (I ; Z=2H)

On fait réagir la tryptamine (II) avec l'acide oxo-2 - pentanedioïque (III) en milieu aqueux chlorhydrique, pour obtenir un composé (I) dans lequel Z est O. Quand on réduit celui-ci, par exemple à l'aide de $AlCl_3$ et $LiAlH_4$, on obtient un composé (I) dans lequel Z représente deux atomes d'hydrogène.

Schéma 2

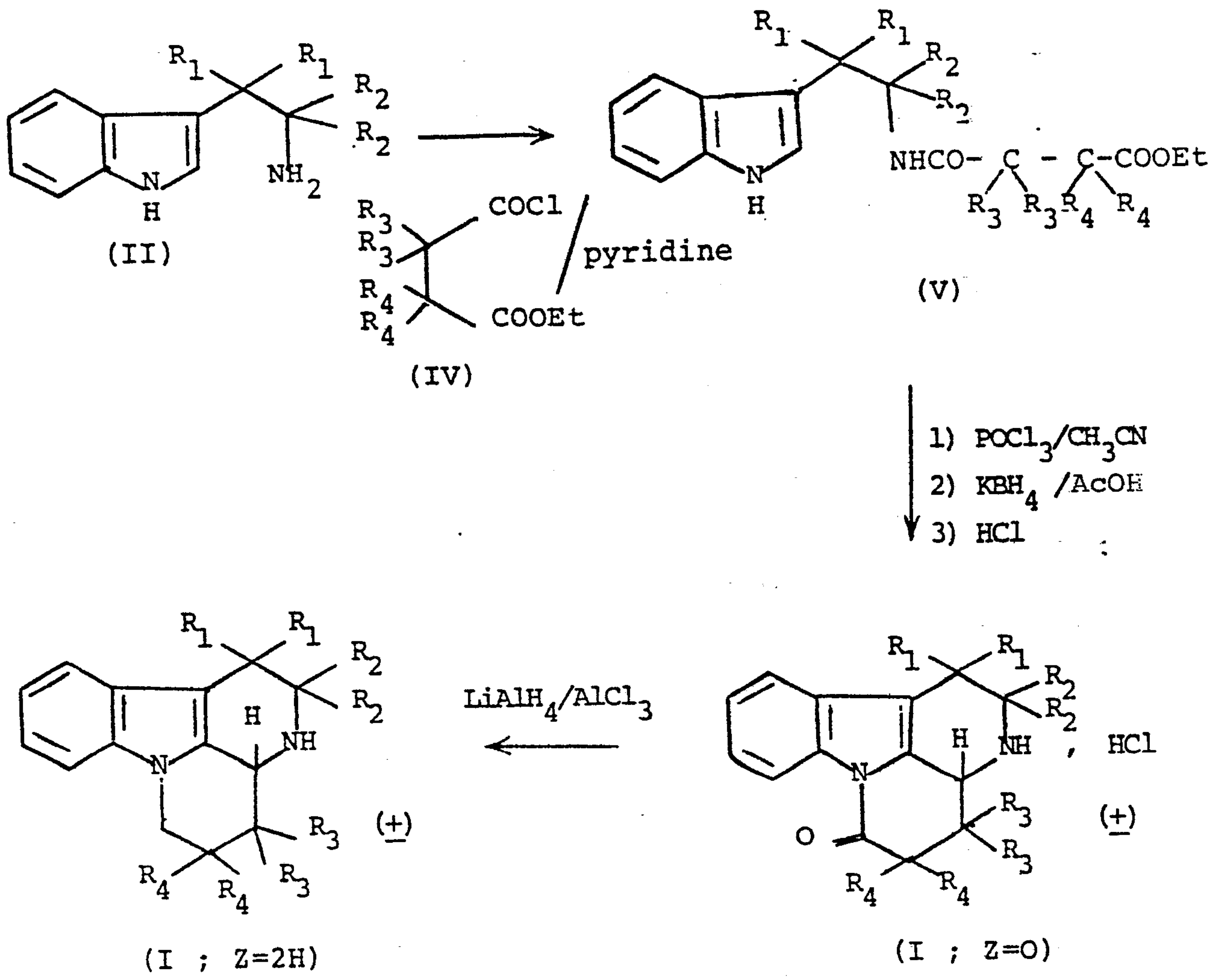


On fait réagir la tryptamine (II) avec le chloro-4 oxo-4 butanoate d'éthyle (IV) en présence de pyridine, puis on effectue la double cyclisation du composé (V) d'abord à l'aide de $POCl_3$ puis par addition de $KBH_4$/AcOH et enfin par addition d'acide chlorhydrique 4N. On obtient un composé (I) dans lequel Z est O. On peut le réduire par exemple à l'aide de $AlCl_3$ et $LiAlH_4$ pour obtenir un composé (I) dans lequel Z représente deux atomes d'hydrogène.

Au lieu de la tryptamine primaire, de formule II, on peut également utiliser la tryptamine protégée par un groupe benzyle ($-NH-CH_2-C_6H_5$ au lieu de $-NH_2$). Ce groupe benzyle doit alors être éliminé de la position 3 en fin de processus, par exemple par hydrogénation catalytique.

Les exemples suivants illustrent l'invention.
Les analyses et les spectres IR et RNM ont confirmé la structure des composés.

**EXEMPLE 1** Diméthyl-2,2 hexahydro-1,2,3,3a,4,5 6H-indolo [3,2,1-de] [naphtyridine-1,5] one-6 et son chlorhydrate.

1. N-[(1H-indolyl-3)-2 diméthyl-1,1 éthyl] amino -4 oxo-4 butanoate d'éthyle.

On met dans un ballon 27,2 ml de chloro-4 oxo-4 butanoate d'éthyle, et 50 ml de $CH_2Cl_2$. On agite et on ajoute 30 g de α, α -diméthyl-tryptamine en solution dans 500 ml de $CH_2Cl_2$ et 13,35 ml de pyridine, en cinq minutes. On agite à la température ambiante pendant une heure. On ajoute 150 ml d'acide chlorhydrique 1N et on agite. On sépare la phase organique et on la lave avec 2x150 ml d'acide chlorhydrique 1N. On sèche la phase organique puis on chasse le solvant. On obtient une huile que l'on chromatographie sur silice. Après élution avec un mélange $CH_2Cl_2$/acétone (8:2), on obtient le [[(1H-indolyl-3)-2 diméthyl-1,1 éthyl] amino]-4 oxo-4 butanoate d'éthyle, que l'on utilise directement dans l'étape suivante.

2. Diméthyl-2,2 hexahydro-1,2,3,3a,4,5 6H - indolo [3,2,1-de] [naphtyridine-1,5] one-6 et son chlorhydrate.

On met dans un ballon 28 g du composé obtenu sous 1 et on ajoute 100 ml de $CH_3CN$. On ajoute 6,46 ml de $POCl_3$ et on chauffe à la température du reflux pendant 2 heures. On chasse le $CH_3CN$ et ajoute 100 ml d'éthanol. On chasse l'éthanol et ajoute un mélange de 200 ml d'éthanol et 40 ml d'acide acétique. On ajoute 4,4 g de $KBH_4$ et agite pendant 30mn. On chasse le solvant, ajoute 200 ml d'eau et laisse reposer à la température ambiante pendant une nuit.

On ajoute 500 ml de $CH_2Cl_2$ et on alcalinise avec de l'ammoniaque diluée. On sépare la phase organique et on extrait la phase aqueuse avec 2 x 200 ml de $CH_2Cl_2$. On réunit les extraits et chasse le solvant. On obtient une huile que l'on utilise directement dans l'étape suivante.

A 28,1 g de cette huile on ajoute 500 ml d'acide chlorhydrique 4N et 50 ml d'éthanol. On chauffe à la température de reflux pendant 6 heures et refroidit dans un bain de glace. On alcalinise avec de l'ammoniaque. On extrait la phase organique avec de l'acétate d'éthyle, sèche les extraits organiques et chasse le solvant. On obtient une huile que l'on chromatographie sur silice. Après élution à l'aide d'acétate d'éthyle puis d'un mélange $CHCl_3$/EtOH (8 : 2) on obtient une huile à laquelle on ajoute 50 ml d'éther. On fait barbotter du gaz chlorhydrique. On essore et sèche sous vide le précipité formé qui est le chlorhydrate du composé.

F > 260°C

EXEMPLE 2 Diméthyl-2,2 hexahydro-2,3,3a,4,5,6 1H-indolo [3,2,1-de] [naphtyridine-1,5] et son méthanesulfonate.

A une solution de 4,1 g d'$AlCl_3$ dans 38 ml d'éther, on ajoute 1,73 g de $AlLiH_4$. Après agitation on ajoute 25 ml de tétrahydrofuranne (THF) et peu à peu 4,4 g du composé obtenu selon l'exemple 1. On ajoute encore 25 ml de THF et agite à la température ambiante pendant une heure. On refroidit dans un bain de glace. On ajoute 7,5 ml d'eau, 7,5 ml de lessive de soude, 750 ml d'eau puis 200 ml d'acétate d'éthyle et on filtre. On sépare la phase organique, extrait la phase aqueuse avec 3 fois 150 ml d'acétate d'éthyle. On chasse ce dernier et on obtient une huile à laquelle on ajoute 100 ml d'éther. On ajoute à la solution 1,6 g d'acide methanesulfonique en solution dans 15 ml d'éthanol, on obtient un solide blanc que l'on recristallise dans de l'éthanol.

F = 226-7°C

EXEMPLE 3 Diméthyl-5,5 hexahydro-1,2,3,3a,4,5 6H-indolo [3,2,1-de] [naphtyridine-1,5]one-6 et son chlorhydrate.

On fait réagir 26,6 g d'acide dimethyl-2,2 oxo-4 pentane-dioïque et 24,47 g de tryptamine, en solution dans 390 ml d'acide chlorhydrique 4N. On porte à la température du reflux, tout en agitant pendant 7h30, puis laisse reposer une nuit à la température ambiante.
On alcalinise par de l'ammoniaque diluée puis on ajoute 1,2 l d'acétate d'éthyle. On décante la phase organique et on chasse l'acétate d'éthyle. Après chromatographie sur silice avec élution par un mélange 95/5 de $CH_2Cl_2/CH_3OH$ on obtient un solide que l'on met en solution dans 150 ml d'éther, on ajoute lentement 50 ml d'éther chlorhydrique. Après agitation, filtration et séchage sous vide sur $P_4O_{10}$, le composé fond à 220°C (déc).

EXEMPLE 4 Diméthyl-5,5 hexahydro-2,3,3a,4,5,6 1H-indolo [3,3,1-de] [naphtyridine-1,5] et son méthanesulfonate.

Dans un ballon contenant 1,4 g d'$AlCl_3$, 22 ml d'éther, 0,65 g de $AlLiH_4$ et 22 ml de THF, on ajoute lentement à la température ambiante, en agitant, 1,63 g du composé obtenu selon l'exemple 3.
On maintient le mélange réactionnel à la température ambiante pendant une heure puis le refroidit dans un bain de glace.
On ajoute 50 ml d'eau, 1 ml de NaOH concentrée et 100 ml d'acétate d'éthyle. On agite le mélange réactionnel puis filtre. On reprend le filtrat et on sépare la phase organique. On chasse le solvant et on obtient des cristaux.
On dissout 1,17 g de la base obtenue dans 30 ml d'isopropanol. On ajoute une solution de 0,52 g d'acide methanesulfonique dans 5 ml d'acétate d'éthyle. On obtient un solide qui après recristallisation dans de l'isopropanol fond à 218°C.

EXEMPLE 5 Diméthyl-1,1 hexahydro-1,2,3,3a,4,5 6*H* -indolo [3,2,1-*de*] [naphtyridine-1,5]one-6 et son chlorhydrate.

On fait réagir 1,88 g (0,01 mole de β,β -diméthyl-tryptamine en solution dans 20 ml d'acide chlorhydrique 3 N avec 1,7 g (0,012 mole) d'acide oxo-2 pentanedioïque.

On chauffe le mélange réactionnel à 110°C pendant 4h, on filtre le chlorhydrate qui précipite, le sèche et le fait recristalliser dans de l'éthanol.

F > 300°C

EXEMPLE 6 Diméthyl-1,1 hexahydro-2,3,3a,4,5,6 1*H*-indolo [3,2,1-*de*] [naphtyridine-1,5] et son méthanesulfonate.

Dans un ballon contenant 15 ml de THF, 0,53 g de $AlCl_3$ et 0,23 g de $AlLiH_4$, on ajoute, par petites quantités, 0,58 g (0,002 mole) du composé obtenu précédemment. On maintient le mélange réactionnel à la température ambiante pendant 1h. On ajoute 3,1 ml de soude, puis 50 ml d'eau et 50 ml d'acétate d'éthyle. On agite le mélange réactionnel, puis filtre, décante, lave à l'eau, sèche sur $Na_2SO_4$ et évapore à sec. On dissout la base obtenue dans un minimun d'éthanol et ajoute 0,16 g (1,6mmole) d'acide méthanesulfonique. Le sel précipite et est recristallisé dans de l'éthanol.

F = 212 - 214°C

EXEMPLE 7 Diméthyl-4,4 hexahydro-1,2,3,3a,4,5 6H-indolo [3,2,1-de] [naphtyridine-1,5] et son chlorhydrate.

1. N-[(1H-indolyl-3)-2 éthyl] N-benzyl-amino-4 oxo-4 diméthyl-3,3 butanoate d'éthyle.

Dans un ballon de 1 l équipé d'une ampoule isobare, d'un balayage à l'azote, d'un réfrigérant, d'une garde à chlorure de calcium, d'un agitateur magnétique et d'un chauffage à bain d'huile, on place 30,06 g (0,12 mole) de (benzylamino-2 éthyl)-3 indole, 200 ml de tétrahydrofuranne anhydre, 11,07 g, soit 12,28 ml (0,14 mole) de pyridine, puis on ajoute lentement, à température ambiante, 26,83 g (0,14 mole) de chloro-4 oxo-4 diméthyl-3,3 butaonate d'éthyle. Ensuite on chauffe au reflux pendant 7 heures, on laisse refroidir, on sépare l'insoluble par filtration, et on concentre le filtrat. On reprend l'huile brune obtenue avec 200 ml d'acétate d'éthyle, on y ajoute 200 ml d'acide chlorydrique 1 N, on sépare la phase organique, on la lave à l'eau, puis par 100 ml de solution à 5 % de bicarbonate de sodium, puis de nouveau à l'eau, on la sèche sur $MgSO_4$ et on la concentre en l'évaporant. Il reste 40,66 g d'une huile brune que l'on purifie par chromatographie sur silice en éluant avec un mélange 98/2 de chlorure de méthylène/acétate d'éthyle.

On obtient 14,5 g de cristaux ocre que l'on utilise tels quels.

2. Diméthyl-4,4 benzyl-3 hexahydro-1,2,3,3a,4,5 6H-indolo [3,2,1-de] [naphtyridine] one-6.

Dans le même appareil que le précédent on place les 14,5 g des cristaux obtenus, 120 ml d'acétonitrile anhydre et 27,3 g, soit 16,4 ml d'oxychlorure de phosphore. On chauffe au reflux durant 8 heures et on laisse refroidir.

On concentre le mélange, on le verse dans un bêcher de 1l, on ajoute 50 ml d'eau, 50 ml d'éthanol, on le refroidit à 0°C, on le rend basique par de l'ammoniaque concentrée et on ajoute par portions 12 g de borohydrure de potassium. On agite 30 mn à température ambiante, puis on ajoute 200 ml d'eau et 100 ml d'acétate d'éthyle, on sépare la phase organique, on la lave à l'eau et on la sèche sur $MgSO_4$. En l'évaporant on recueille 11,3 g d'une huile jaune épaisse. On purifie cette dernière par chromatographie sur silice en éluant avec un mélange 98/2 de chlorure de méthylène/méthanol.

On recueille ainsi 5,91 g d'une huile jaune que l'on utilise telle quelle pour la suite.

3. Diméthyl-4,4 benzyl-3 hexahydro-1,2,3,3a,4,5 6<u>H</u>-indolo [3,2,1-de] [naphtyridine] .

Dans le même appareil que le précédent on place 5,26 g de $AlCl_3$, 80 ml de tétrahydrofuranne, 2,3 g de $AlLiH_4$ et on ajoute lentement 5,91 g de l'huile précédemment obtenue dissoute dans 60 ml de tétrahydrofuranne. On porte ensuite le mélange lentement à la température du reflux, on le laisse 4 heures à cette température, et on laisse reposer une nuit. Pour être certain d'épuiser tout le produit de départ, on ajoute encore 1 g de $AlLiH_4$ et on remet au reflux pendant 2 heures. Après refroidissement à 0°C, on ajoute lentement 200 ml d'eau, 10 ml d'ammoniaque concentrée, et 100 ml d'acétate d'éthyle. On filtre pour éliminer les produits minéraux, on sépare la phase organique, on la lave, on la sèche et on l'évapore sous vide.

On obtient 4,71 g d'une huile jaune que l'on purifie par chromatographie sur silice en éluant par le chlorure de méthylène.

On recueille ainsi 0,81 g d'une huile que l'on utilise telle quelle pour la suite.

4. Diméthyl-4,4 hexahydro-1,2,3,3a,4,5 6H-indolo [3,2,1-de] [naphtyridine] .

Dans un flacon de Parr de 250 ml on place 0,5 g de l'huile précédemment obtenue, avec 20 ml d'éthanol, 4 ml d'acide acétique et 0,05 g de charbon palladié à 10 %.
On effectue une hydrogénation à 50°C sous environ 0,35 MPa pendant 1 heure. Ensuite on sépare le catalyseur par filtration, on évapore le solvant, on ajoute 30 ml d'eau à l'huile résiduelle, puis 10 ml d'ammoniaque à 10 %, et 50 ml d'acétate d'éthyle. On sépare les phases, on lave et on sèche la phase organique, et on l'évapore sous vide. Il reste 0,37 g de cristaux orangés que l'on reprend par 15 ml d'acétate d'éthyle puis, sur bain de glace, on ajoute lentement 5 ml d'éther chlorhydrique. On agite le précipité formé pendant 1 heure à 0°C, on le filtre sur verre fritté, on le lave à l'acétate d'éthyle et on le sèche sous vide à 80°C. On recueille ainsi 0,31 g de chlorhydrate.

F = 258-259°C.

Les composés de l'invention préparés à titre d'exemples sont représentés dans le tableau suivant

TABLEAU

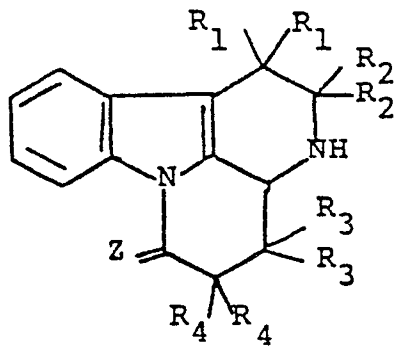

(I)

| Composé | $R_1$ | $R_2$ | $R_3$ | $R_4$ | Z | Sel | F(°C) |
|---|---|---|---|---|---|---|---|
| 1 | H | $CH_3$ | H | H | O | HCl | > 260 |
| 2 | H | $CH_3$ | H | H | HH | $CH_3SO_3H$ | 226-227 |
| 3 | H | H | H | $CH_3$ | O | HCl | 220 |
| 4 | H | H | H | $CH_3$ | HH | $CH_3SO_3H$ | 218 |
| 5 | $CH_3$ | H | H | H | O | HCl | >300 |
| 6 | $CH_3$ | H | H | H | HH | $CH_3SO_3H$ | 212-214 |
| 7 | H | H | $CH_3$ | H | HH | HCl | 258-259 |

0130878

Les composés de l'invention ont fait l'objet d'une étude pharmacologique.

1. TOXICITE

La dose létale 50 (DL50) des composés est déterminée chez des souris de souche CD1 par méthode graphique. La DL50 va de 30 à 100 mg/kg par voie i.p.

2. HYPOXIE HYPOBARE

Des souris de souche CD1 sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygènation tissulaire et en particulier cérébrale. Les composés étudiés sont administrés, à plusieurs doses, par voie intrapéritonale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement. La DAM est de 2 à 10 mg/kg i.p. environ.

3. ISCHEMIE CEREBRALE COMPLETE

Les composés de l'invention ont été soumis au test de l'ischémie cérébrale complète chez la souris. L'ischémie est due à un arrêt cardiaque induit par une injection intraveineuse rapide de $MgCl_2$. Dans ce test on mesure le "temps de survie", c'est-à-dire l'intervalle entre le moment de l'injection de $MgCl_2$ et le dernier mouvement inspiratoire observable de chaque souris.
Ce dernier mouvement est considéré comme l'indice ultime d'une fonction du système nerveux central.
L'arrêt respiratoire apparaît approximativement 19 secondes après l'injection de $MgCl_2$.

Des souris mâles (Charles River CD1) sont étudiées par groupes de 10.
Les souris sont nourries et abreuvées ad libitum avant les essais. Le temps de survie est mesuré 10 minutes après l'administration intrapéritonéale des composés de l'invention.
Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.
Les rapports entre les modifications dans le temps de survie et la dose du composé sont enregistrés graphiquement selon une courbe semilogarithmique.
Cette courbe permet le calcul de la dose effective 3 secondes ($DE_{3"}$), c'est-à-dire la dose (en mg/kg) qui produit une augmentation de 3 secondes du temps de survie par rapport au groupe témoin de 10 souris non traitées.
Une augmentation de 3 secondes dans le temps de survie est à la fois significative statistiquement et reproductible.

La $DE_{3"}$ des composés de l'invention varie de 2,2 à 7,4 mg/kg par voie i.p.

L'étude pharmacologique des composés de l'invention montre qu'ils sont actifs dans l'épreuve d'hypoxie hypobare chez la souris tout en n'étant que peu toxiques.

Les composés de l'invention possèdant une activité antianoxique et anti-ischémique peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, pour le traitement des encéphalopathies métaboliques.

L'invention comprend par conséquent, toutes compositions pharmaceutiques renfermant les composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 10 à 100 mg.

Revendications pour les états contractants : BE, CH, DE, FR, GB, IT, LI, LU, NL, SE.

1. Dérivés d'hexahydro-indolo-naphtyridines, sous la forme de racémates ou d'énantiomères, répondant à la formule (I)

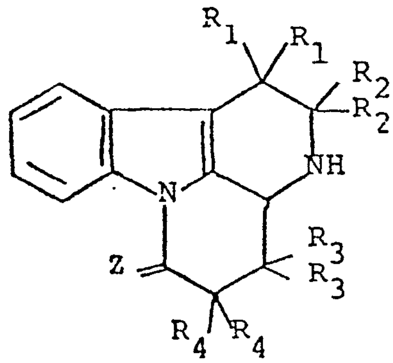

(I)

dans laquelle
Z représente un atome d'oxygène ou 2 atomes d'hydrogène,
$R_1$ ou $R_2$ ou $R_3$ ou $R_4$ représente le radical méthyle tandis que les autres sont des atomes d'hydrogène, ainsi que leurs sels d'addition aux acides pharmaceutiquement acceptables.

2. Dérivés selon la revendication 1, dans lesquels Z représente un atome d'oxygène.

3. Dérivés selon la revendication 1, dans lesquels Z représente deux atomes d'hydrogène.

4. Procédé de préparation des composés selon la revendication 1 procédé caractérisé en ce que l'on fait réagir une tryptamin de formule (II)

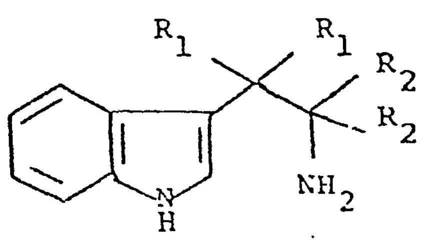

(II)

avec un diacide de formule (III)

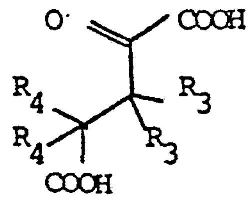

(III)

en milieu aqueux chlorhydrique pour obtenir un composé (I) dans lequel Z représente un atome d'oxygène puis, si on le désire, on réduit ce composé pour obtenir un composé (I) dans lequel Z représente deux atomes d'hydrogène.

5. Procédé de préparation des composés selon la revendication 1, procédé caractérisé en ce que l'on fait réagir une tryptamine de formule (II)

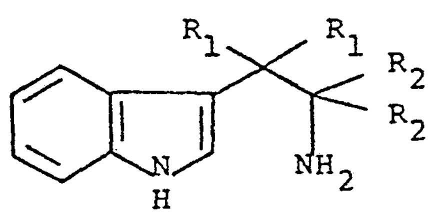

(II)

dans laquelle le groupe $NH_2$ est éventuellement benzylé, avec un chlorure d'acide de formule (IV)

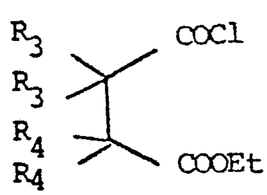

(IV)

pour obtenir un composé de formule (V)

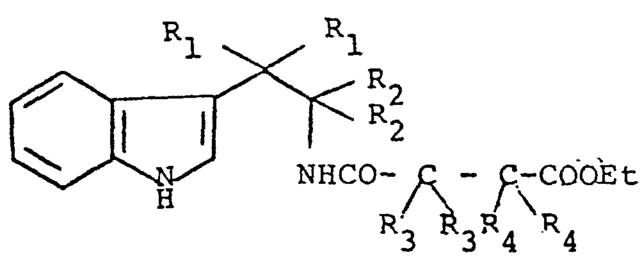

(V)

que l'on cyclise en composé (I) dans lequel Z représente un atome d'oxygène puis, si on le désire, on réduit ce composé pour obtenir le composé (I) dans lequel Z représente deux atomes d'hydrogène et, le cas échéant, on élimine le groupe benzyle de la position 3.

6. Procédé selon la revendication 4 ou la revendication 5, caractérisé par le fait que la réduction est effectuée à l'aide de chlorure d'aluminium et d'hydrure double de lithium et d'aluminium.

7. Médicament caractérisé en ce qu'il contient un composé selon l'une quelconque des revendications 1 à 3.

8. Composition pharmaceutique caractérisée en ce qu'elle contient un composé selon l'une quelconque des revendications 1 à 3 en association avec tout excipient approprié.

Revendications pour l'état contractant : AT.

1.Procédé de préparation de composés répondant à la formule (I)

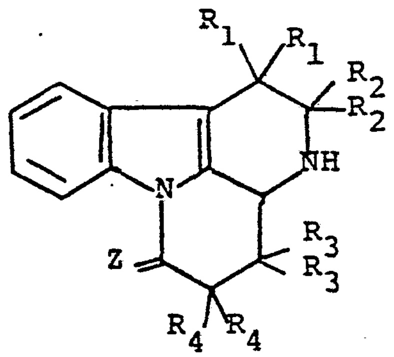

(I)

dans laquelle

Z représente un atome d'oxygène ou 2 atomes d'hydrogène,

$R_1$ ou $R_2$ ou $R_3$ ou $R_4$ représente le radical méthyle tandis que les autres sont des atomes d'hydrogène,

ainsi que de leurs sels d'addition aux acides pharmaceutiquement acceptables,

procédé caractérisé en ce que l'on fait réagir une tryptamine de formule (II)

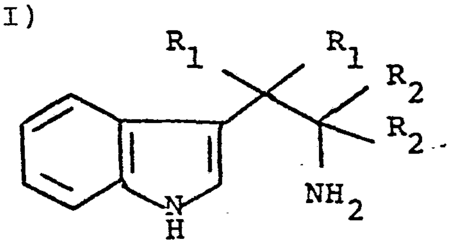

(II)

avec un diacide de formule (III)

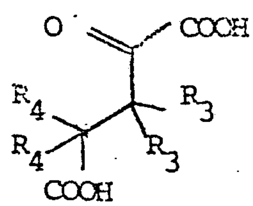

(III)

en milieu aqueux chlorhydrique pour obtenir un composé (I) dans lequel Z représente un atome d'oxygène puis, si on le désire, on réduit ce composé pour obtenir un composé (I) dans lequel Z représente deux atomes d'hydrogène.

2. Procédéde préparation des composés de formule (I) tels que définis dans la revendication 1, procédé caractérisé en ce que l'on fait réagir une tryptamine de formule (II)

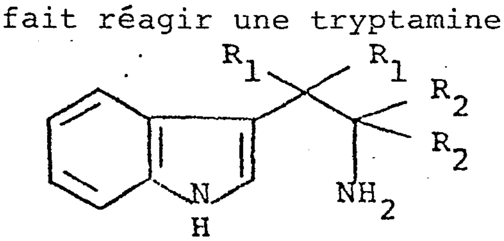
(II)

dans laquelle le groupe $NH_2$ est éventuellement benzylé, avec un chlorure d'acide de formule (IV)

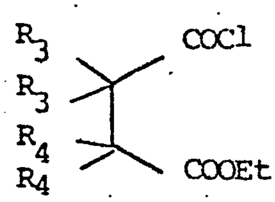
(IV)

pour obtenir un composé de formule (V)

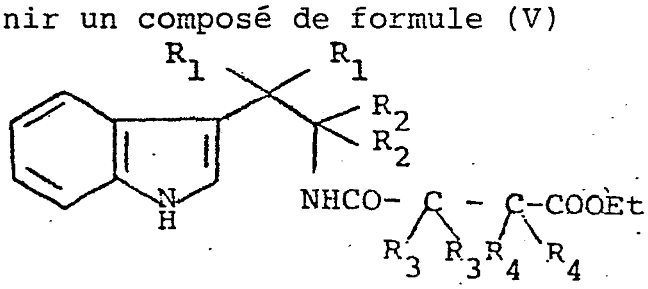
(V)

que l'on cyclise en composé (I) dans lequel Z représente un atome d'oxygène puis, si on le désire, on réduit ce composé pour obtenir le composé (I) dans lequel Z représente deux atomes d'hydrogène et, le cas échéant, on élimine le groupe benzyle de la position 3.

3. Procédé selon la revendication 1 ou la revendication 2 caractérisé par le fait que la réduction est effectuée à l'aide de chlorure d'aluminium et d'hydrure double de lithium et d'aluminium.

0130878

Office européen des brevets

# RAPPORT DE RECHERCHE EUROPEENNE

Numéro de la demande

EP 84 40 1255

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-2 376 150 (J. LOGEAIS)<br>* revendication 1 *<br>--- | 1,7 | C 07 D 471/16<br>A 61 K 31/435//<br>C 07 D 209/16<br>(C 07 D 471/16<br>C 07 D 221/00<br>C 07 D 221/00<br>C 07 D 209/00 ) |
| A | FR-A-2 374 905 (SYNTHELABO)<br>* revendication 1; pages 8-11 *<br>----- | 1,7 | |
| | | | DOMAINES TECHNIQUES RECHERCHES (Int. Cl. 3) |
| | | | C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 12-09-1984 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82